(19) Europäisches Patentamt
European Patent Office
Office européen des brevets

(11) Veröffentlichungsnummer: **0 208 147**
**A2**

# (12) EUROPÄISCHE PATENTANMELDUNG

(21) Anmeldenummer: **86107710.5**

(22) Anmeldetag: **15.02.84**

(51) Int.Cl.⁴: **C 07 D 311/58**
C 07 D 311/70, C 07 D 311/64
C 07 D 493/04, A 01 N 43/16

(30) Priorität: **15.02.83 HU 50483**

(43) Veröffentlichungstag der Anmeldung:
**14.01.87 Patentblatt 87/3**

(84) Benannte Vertragsstaaten:
**AT BE CH DE FR GB IT LI NL SE**

**⁷⁄₈¼Veröffentlichungsnummer der früheren Anmeldung nach Art. 76 EPÜ: 0 118 794**

(71) Anmelder: **ALKALOIDA VEGYéSZETI GYáR**
**Postfach 1**
**H-4440 Tiszavasvári(HU)**

(72) Erfinder: **Timar, Tibor, Dr. Dipl.-Ing.**
**Elmunkás u. 27**
**H-4440 Tiszavasvári(HU)**

(72) Erfinder: **Zsupán, Kálmán, Dr. Dipl. Ing.**
**Elmunkás u. 23/a**
**H-4440 Tiszavasvári(HU)**

(72) Erfinder: **Répási, Janos, Dr.**
**Elmunkás u. 1**
**H-4440 Tiszavasvári(HU)**

(72) Erfinder: **Borsos geb. Safranek, Irén**
**Elmunkás u. 1**
**H-4440 Tiszavasvári(HU)**

(72) Erfinder: **Kiss, Istvén, Dr.**
**Zöldfa u. 4**
**H-6723 Szeged(HU)**

(72) Erfinder: **Fodor, Andás, Dr.**
**Retek u. 2**
**H-6723 Szeged(HU)**

(72) Erfinder: **Maróy, Péter, Dr.**
**Ürhajós u. 5/a**
**H-6723 Szeged(HU)**

(74) Vertreter: **Patentanwälte Beetz sen. - Beetz jun. Timpe - Siegfried - Schmitt-Fumian**
**Steinsdorfstrasse 10**
**D-8000 München 22(DE)**

## (54) **Neue Chromenderivate, ihre Herstellung und ihre Verwendung zur Schädlingsbekämpfung.**

(57) Die Erfindung betrifft Precocen-Analoga der Formel I

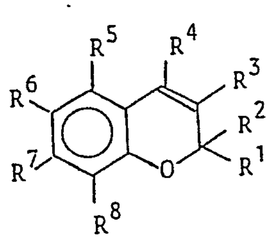

(I),

worin bedeuten:
$R^1$ und $R^2$ H oder $C_{1-4}$-Alkyl,
$R^3$ und $R^4$ H oder Halogen,
$R^5$ H, $C_{1-10}$-Alkyl oder $C_{1-6}$-Alkoxy,
$R^6$ H, ggfs. substituiertes $C_{1-6}$-Alkoxy, $C_{2-3}$-Alkylendioxy oder Aralkyloxy,
$R^7$ ggfs. substituiertes $C_{2-3}$-Alkoxy, $C_{5-8}$-Cycloalkyloxy oder ggfs. substituiertes Aralkyloxy, Dialkylaminoalkoxy oder Cycloalkylaminoalkyloxy,
wobei $R^6$ und $R^7$ zusammen auch $C_{2-8}$-Alkylenoxy-alkylenoxy bedeuten können und
$R^8$ Wasserstoff, $C_{1-6}$-Alkoxy oder Aralkoxy oder $C_{1-6}$-Alkyl, wobei $R^7$ und $R^8$ zusammen auch $C_{2-8}$-Alkylenoxy bedeuten können,
mit der Maßgabe, daß, wenn $R^6$ Methoxy und $R^7$ Ethoxy- oder Isopropyloxy darstellen, $R^3$, $R^4$ und $R^5$ nicht gleichzeitig Wasserstoff bedeuten können,
Verfahren zu ihrer Herstellung sowie ihre Verwendung in Schädlingsbekämpfungsmitteln.

EP 0 208 147 A2

Croydon Printing Company Ltd

0208147

NEUE CHROMENDERIVATE, IHRE HERSTELLUNG UND IHRE VERWENDUNG ZUR SCHÄDLINGSBEKÄMPFUNG

Die Erfindung betrifft neue Derivate des 7-Oxy-2H-chromens der allgemeinen Formel I,

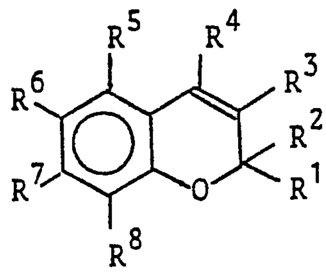

(I),

worin bedeuten:

$R^1$ und $R^2$ Wasserstoff oder $C_{1-4}$-Alkyl,

$R^3$ und $R^4$ Wasserstoff oder Halogen,

$R^5$ Wasserstoff, $C_{1-10}$-Alkyl oder $C_{1-6}$-Alkoxy,

$R^6$ Wasserstoff, $C_{1-6}$-Alkoxy, das gesättigt oder ungesättigt und gegebenenfalls mit einer Trihalogenmethylgruppe substituiert ist, $C_2$- oder $C_3$-Alkylendioxy oder Aralkyloxy,

$R^7$ $C_{2-3}$-Alkoxy, das gesättigt oder ungesättigt und gegebenenfalls mit einer $C_{1-6}$-Alkyl-, $C_{1-6}$-Alkylthio-, $C_{1-6}$-Alkoxy-, Trihalogenmethyl- oder Hydroxylgruppe substituiert ist, $C_{5-8}$-Cycloalkyloxy oder Aralkyloxy, das gegebenenfalls mit einem Halogenatom substituiert ist, Dialkylaminoalkoxy

oder Cycloalkylaminoalkyloxy,

wobei $R^6$ und $R^7$ zusammen auch $C_{2-8}$-Alkylenoxy-alkylenoxy bedeuten können,

und

$R^8$ Wasserstoff, $C_{1-6}$-Alkoxy oder Aralkoxy oder $C_{1-6}$-Alkyl,

wobei $R^7$ und $R^8$ zusammen auch $C_{2-8}$-Alkylenoxy-alkylenoxy bedeuten können,

mit der Maßgabe, daß, wenn $R^6$ Methoxy und $R^7$ Ethoxy- oder Isopropyloxy darstellen, $R^3$, $R^4$ und $R^5$ nicht gleichzeitig Wasserstoff bedeuten können,

und ihre Salze,
ferner Verfahren zur Herstellung dieser Verbindungen sowie Schädlingsbekämpfungsmittel mit Verbindungen der Formel I als Wirkstoffen.

Die Verfahren gemäß der Erfindung zur Herstellung der 2H-Chromenderivate der Formel I mit $R_3$, $R_4$ = H, d.h. von 2H-Chromenderivaten der Formel IA,

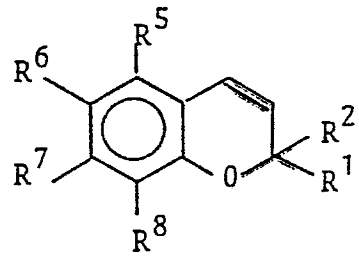

(IA),

worin $R^1$, $R^2$ und $R^5$ - $R^8$ die obige Bedeutung besitzen,

sind durch folgende Verfahrensweisen gekennzeichnet:

(a) Dehydratisierung von Chromanolderivaten der allgemeinen Formel

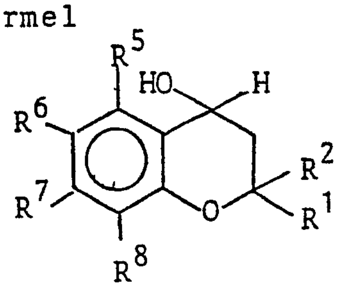

(III)

mit $R^1$, $R^2$ und $R^5$ - $R^8$ wie oben;

(b) Umsetzung von Cumarinderivaten der Formel IV

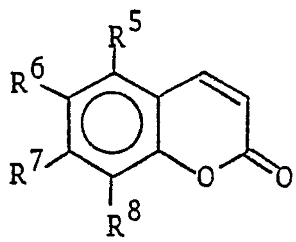

(IV)

mit $R^5$ - $R^8$ wie oben

mit Grignard-Reagentien der Formel

$R^1MgX$ und/oder $R^2MgX$,

worin $R^1$ und $R^2$ die obige Bedeutung besitzen, und

X Halogen darstellt;

(c) Dehydratisierung von Chinonalliden der allgemeinen Formel VI

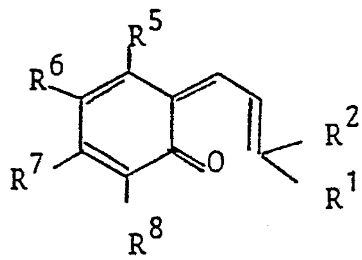

(VI)

mit $R^1$, $R^2$ und $R^5$- $R^8$ wie oben,

vorzugsweise in aromatischen Lösungsmitteln bei 60 bis 120 °C mit Kaliumdichromat;

(d) Dehydrohalogenierung von Chromanderivaten der allgemeinen Formel VII

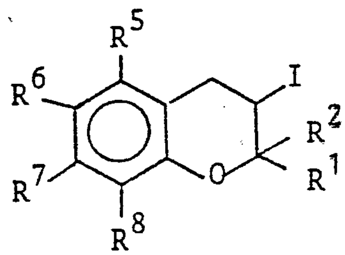

(VII)

mit $R^1$, $R^2$ und $R^5$ - $R^8$ wie oben,

vorzugsweise in alkoholisch/basischem Medium beim Siedepunkt der Alkohole;

(e) Cyclisierung von Ether der allgemeinen Formel X

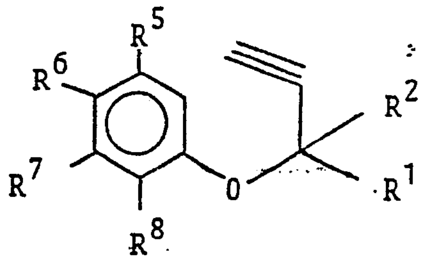

(X)

mit $R^1$, $R^2$ und $R^5$ - $R^8$ wie oben,

vorzugsweise in N,N-Dialkylarylaminen bei 160 bis 250 °C;

(f) Umsetzung von Phenolen der allgemeinen Formel VIII

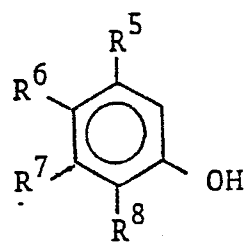

(VIII)

mit $R^5$ - $R^8$ wie oben

mit Carbonylverbindungen der allgemeinen Formel XI

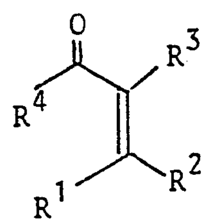

(XI)

mit $R^1$ - $R^4$ wie oben

oder deren geschützten Derivaten, vorzugsweise in aromatischen aprotischen Lösungsmitteln in Form ihrer entsprechenden Schwermetallsalze bei 100 bis 150 °C

oder

(g) Oxidation von Chromanen der allgemeinen Formel XIII

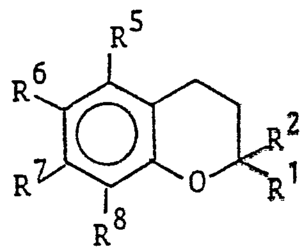

(XIII)

mit $R^1$, $R^2$ und $R^5$ - $R^8$ wie oben.

Die erfindungsgemäße Verfahrensweise zur Herstellung von 2H-Chromenderivaten der allgemeinen Formel I mit $R^3$, $R^4$ = Halogen, d.h. von 2H-Chromenderivaten der allgemeinen Formel IB

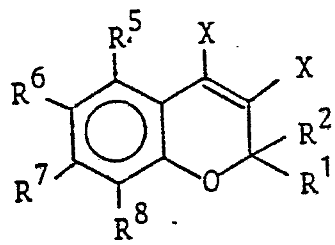

(IB)

mit $R^1$, $R^2$ und $R^5$ - $R^8$ wie oben

und

X = Halogen,

ist dadurch gekennzeichnet, daß

(h) 4-Chromanonderivate der allgemeinen Formel II

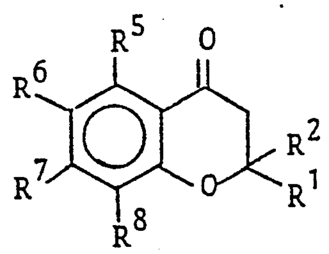

(II)

mit $R^1$, $R^2$ und $R^5$ - $R^8$ wie oben

mit Phosphorpentahalogeniden, vorzugsweise in Kohlenstofftetrachlorid bei 15 bis 25 °C, umgesetzt werden, und anschließend aus der erhaltenen Verbindung ein Salz gebildet oder aus dem Salz die Verbindung der Formel IB freigesetzt wird.

Die abhängigen Ansprüche 3 bis 10 betreffen bevorzugte Ausführungsformen der erfindungsgemäßen Verfahrensweisen.

Die erfindungsgemäßen neuen Verbindungen sind Analoga der Naturstoffe Precocen-1 (P1) und Precocen-2 (P2). Die biologische Wirkung von P1 und P2 auf Insekten ist bekannt. Aufgrund ihrer biologischen Wirkung auf Insekten können diese Verbindungen als neuartige umweltschonende Schädlingsbekämpfungsmittel verwendet werden (Science 193 (1976) 542). Die biologische Wirksamkeit hängt im Prinzip von der Art der Schädlinge und den angewandten Testmethoden bzw. von der Art und Anzahl der Substituenten des aromatischen Ringes und ihrer Stellung usw. ab (Science 197 (1977) 1369; Experientia 35 (1979) 363; Z. Naturforsch. 35b, 1449 (1980); Pestic. Sci. 12/3/ (1981) 245).

Aufgrund früherer Forschungen wurden verschiedene Derivate gefunden, die aktiver als die in der Natur vorkommenden Precocene P1 und P2 sind (z.B. DE-PS 2 639 671; US-PS 4 162 326; JP-PSen 73121/79, 15411/80, 40637/80 und 43039/80; ES-PSen 496 301 und 496 302).

Die Erfindung beruht auf der Feststellung, daß die neuen Chromenderivate der allgemeinen Formel I eine sehr wertvolle Anti-Juvenilhormon-Wirkung (AJH-Wirkung) sowie insecticide und nematocide Wirkung aufweisen.

Die Verfahren zur Herstellung der Verbindungen der allgemeinen Formel umfassen folgende Varianten:

Zur Herstellung von eine Untergruppe der Verbindungen der allgemeinen Formel I bildenden Chromenderivaten der allgemeinen Formel IA werden

(a) Chromanole der allgemeinen Formel III dehydratisiert, oder

(b) Cumarinderivate der allgemeinen Formel IV mit Grignard-Verbindungen der allgemeinen Formel $R^1MgX$ und/oder $R^2MgX$ mit $R^1$, $R^2$ und X wie oben, vorzugsweise in aliphatischen/cycloaliphatischen $C_{2-8}$-Ethern bei 20 bis 80 °C, umgesetzt, oder

(c) Chinonallide der allgemeinen Formel VI, vorzugsweise in aromatischen Lösungsmitteln bei 60 bis 120 °C mit Kaliumdichromat, dehydratisiert, oder

(d) Chromane der allgemeinen Formel VII, vorzugsweise in alkoholisch/basischem Medium beim Siedepunkt der Alkohole, dehydrohalogeniert, oder

(e) Ether der allgemeinen Formel X, vorzugsweise in N,N-Dialkyl-arylaminen bei 160 bis 250 °C, cyclisiert, oder

(f) Phenole der allgemeinen Formel VIII mit Carbonylverbindungen der allgemeinen Formel XI oder deren geschützten Derivaten, vorzugsweise in aprotischen Lösungsmitteln in Form ihrer entsprechenden Salze bei 100 bis 150 °C, umgesetzt, oder

(g) Chromane der allgemeinen Formel XIII oxidiert.

Zur Herstellung von eine Untergruppe der Verbindungen der allgemeinen Formel I bildenden Chromenderivaten der allgemeinen Formel IB werden

(h) Chromanone der allgemeinen Formel II mit einem Phosphorpentahalogenid, vorzugsweise Phosphorpentachlorid und bevorzugt in Gegenwart von Kohlenstofftetrachlorid bei 15 bis 25 °C umgesetzt.

Aus der erhaltenen Verbindung kann gewünschtenfalls ein Salz hergestellt oder aus dem Salz die freie Verbindung freigesetzt werden.

Gemäß Variante (a) des Verfahrens wird vorteilhaft so verfahren, daß das Chromanol der allgemeinen Formel III mit Dehydratationsmitteln bei

20 bis 160 °C, vorzugsweise mit verdünnter Salzsäure bei 15 bis 25 °C, dehydratisiert wird.

Gemäß Variante (b) des Verfahrens wird als Lösungsmittel vorteilhaft Dimethylether oder Tetrahydrofuran verwendet. Das vorteilhafte Temperaturintervall liegt bei 35 bis 65 °C.

Gemäß Variante (e) des Verfahrens wird die Cyclisierung vorteilhaft in N,N-Dimethylanilin oder N,N-Diethylanilin bei 190 bis 210 °C durchgeführt.

Gemäß Variante (f) des Verfahrens ist es vorteilhaft, das Titansalz des Phenols der allgemeinen Formel XI herzustellen und dieses in Toluol beim Siedepunkt des Reaktionsgemischs umzusetzen.

Gemäß Variante (g) des Verfahrens ist es vorteilhaft, das Chroman der allgemeinen Formel XIII in einem aromatischen Lösungsmittel bei 60 bis 150 °C, vorzugsweise mit Dichlorcyanobenzochinon (DDQ), in Benzol bei 50 bis 80 °C, zu oxidieren.

Gemäß einer vorteilhaften Ausführungsform der Variante (h) des Verfahrens setzt man die 4-Chromanone der allgemeinen Formel II in Gegenwart von partiell halogenierten Alkanen oder Perhalogenalkanen bei 10 bis 15 °C mit Phosphorpentahalogeniden um. Mit Phosphorpentachlorid können sehr gute Ausbeuten erzielt werden.

Die erfindungsgemäßen Schädlingsbekämpfungsmittel

mit insekticider und nematocider sowie Anti-Juvenilhormon-Wirkung enthalten ein festes oder flüssiges Verdünnungsmittel, Füllstoffe, Trägerstoffe, Netzmittel, Dispergiermittel und/oder Emulgiermittel und/oder andere grenzflächenaktive Mittel sowie als Wirkstoff die Verbindungen der allgemeinen Formel I.

Die Erfindung wird durch die folgenden Beispiele näher erläutert.

Die Reinheit der in den Beispielen erläuterten Verbindungen wurde durch Dünnschicht- und Gaschromatographie überprüft. Die Struktur der Verbindungen wurde durch IR-, NMR- und Massenspektrometrie überprüft.

Beispiel für die Angabe von NMR-Spektren:

1,45 - chemische Verschiebung
3H - Anzahl der Protonen
t -- Multiplizität
J - Kopplungskonstante

Multiplett-Bezeichnungen:

s - Singulett
d - Dublett
t - Triplett
q - Quartett
m - größeres Multiplett
sz - breites Signal.

Beispiel 1

6,7-Dibenzyloxy-2,2-dimethyl-2H-chromen

7,8 g (20 mmol) 6,7-Dibenzyloxy-2,2-dimethyl-4-chromanon werden in 100 ml eines Gemischs von Tetrahydrofuran/Wasser (1:1) gelöst und mit 5,3 g (30 mmol) Palladiumchlorid und 8,31 g (220 mmol) Natriumtetrahydroboranat bei 0 °C 3 Stunden lang gerührt. Danach wird der Ansatz abgesaugt, das Filtrat dreimal mit jeweils 100 ml Ether extrahiert und das Lösungsmittel entfernt. Der Rückstand wird in 300 ml Dichlormethan gelöst und mit 15 ml Pyridin und 2 g Phosphoroxychlorid 1 Stunde lang auf 35 °C erwärmt. Der Ansatz wird dann auf 100 ml Wasser gegossen und mit 5 % Salzsäure und mit Wasser gewaschen. Die organische Phase wird getrocknet, das Lösungsmittel entfernt und der Rückstand aus Methanol umkristallisiert. Man erhält 6 g Produkt (Ausbeute 81 %); Fp.: 50 bis 51 °C.

Beispiel 2

3,4-Dichlor-6-methoxy-7-isopropoxy-2,2-dimethyl-2H-chromen

2,64 g (10 mmol) 6-Methoxy-7-isopropoxy-2,2-dimethyl-4-chromanon werden mit 4,2 g (20 mmol) Phosphorpentachlorid und 30 ml Tetrachlorkohlenstoff suspendiert und 8 Stunden lang bei 30 °C gerührt. Der Ansatz wird dann eingeengt und der Rückstand aus Ethanol umkristallisiert. Ausbeute: 3,8 g (60 %); Fp.: 80 bis 85 °C.

Beispiel 3

7-Cyclopentyloxy-2,2,5-trimethyl-2H-chromen

4,6 g (20 mmol) 5-Methyl-7-cyclopentyloxycumarin werden in 70 ml absolutem Ether gelöst und zu 100 ml einer Lösung von Methylmagnesiumjodid (1,5 g Magnesium und 8,5 g Methyljodid) in Ether unter Rühren zugefügt und 5 Stunden lang gekocht. Der Ansatz wird über Nacht stehengelassen und mit 200 ml 5-%iger Ammoniumchloridlösung zersetzt. Nach üblicher Aufarbeitung und Reinigung durch Säulenchromatographie (Kieselgel-60; Hexan/Ether 9:1) erhält man ein farbloses Öl. Ausbeute: 3,8 g (74 %).

Beispiel 4

7-Isobutyloxy-8-methoxy-2,2-dimethyl-2H-chromen

5,8 g (22 mmol) 2-Prenyl-5-isobutyloxy-6-methoxyphenol werden in Gegenwart einer äquivalenten Menge Methyltrialkyl($C_8$-$C_{10}$)-ammoniumdichroman in 100 ml Benzol 1 Stunde lang am Rückfluß gekocht. Der Ansatz wird mit Hexan verdünnt und mit 20-%iger Natriumthiosulfatlösung gewaschen. Die organische Phase wird getrocknet, das Lösungsmittel entfernt und das Produkt durch Säulenchromatographie gereinigt (Beispiel 3). Ausbeute: 4,6 g (80 %); farbloses Öl.

Beispiel 5

5-Methoxy-7-s-butyloxy-2,2-dimethyl-2H-chromen

5,8 g (22 mmol) 2-Prenyl-3-methoxy-5-s-butyloxyphenol werden in 100 ml Dichlormethan gelöst, mit 5 g (22 mmol) N-Jodsuccinimid versetzt und 1 Stunde lang bei 30 °C gerührt. Der Ansatz wird mit 200 ml Wasser verdünnt, die organische Phase abgetrennt, mit 5-%iger Natriumthiosulfatlösung gewaschen, getrocknet und eingeengt. Der Rückstand wird in 100 ml einer 10-%igen Lösung von Natriumhydroxid in Methanol gelöst und 2 Stunden lang auf 50 °C erwärmt. Danach wird mit Wasser verdünnt und mit Ether extrahiert; die organische Phase wird mit 1-%iger Salzsäure und Wasser gewaschen, worauf das Lösungsmittel entfernt und das Produkt analog Beispiel 3 gereinigt wird. Ausbeute: 5,5 g (95 %); hellgelbes Öl.

Beispiel 6

7-n-Propoxy-8-methoxy-2,2-dimethyl-2H-chromen

Eine Suspension aus 5,5 g (30 mmol) 2-Methoxy-3-n-propoxy-phenol, 6,5 g (60 mmol) 3-Chlor-3-methylbut-1-in, 5 g Kaliumcarbonat, 8 g Kaliumjodid und 50 ml absolutem Aceton wird 20 Stunden lang unter Rühren gekocht. Das anorganische Salz wird abgesaugt, der Rückstand nach Einengen des Filtrats in 100 ml N,N-Dimethylanilin 8 Stunden lang gekocht. Der Ansatz wird wie üblich aufgearbeitet und das Produkt analog Beispiel 3 gereinigt. Ausbeute: 5,8 g (80 %); farbloses Öl.

Beispiel 7

7-Ethoxy-2,2,8-trimethyl-2H-chromen

Zu einer Lösung von 5,7 g (25 mmol) Titantetraethoxid in 50 ml Tuoluol wird bei 20 °C unter Stickstoffatmosphäre eine Lösung von 13,8 g (100 mmol) 2-Methyl-3-ethoxyphenol in 50 ml Toluol gegeben. Die orangerote Lösung wird 1 Stunde lang gekocht, dann bis zur Entfernung des Ethanols langsam destilliert. Nach Abkühlung auf Raumtemperatur wird dem Ansatz eine Lösung von 10,6 g (150 mmol) Dimethylacrolein in 200 ml Toluol zugetropft, worauf 8 Stunden lang gekocht wird. Danach wird das Gemisch mit gesättigter Ammoniumchloridlösung verdünnt, mit Ether extrahiert und nach Entfernung des Lösungsmittels analog Beispiel 3 gereinigt. Ausbeute: 10,2 g (50 %); farbloses Öl.

Beispiel 8

5-Methoxy-7-isobutoxy-2,2-dimethyl-2H-chromen

3,9 g (20 mmol) 3-Methoxy-5-isobutoxyphenol und 2,8 g (20 mmol) 1,3-Dichlor-3-methylbutan werden in Gegenwart von 0,26 g (1 mmol) Bis(acetyl-acetonato)-nickel 8 Stunden lang auf 125 °C erwärmt. Der Ansatz wird abgekühlt und mit 25 ml Hexan verdünnt. Nach Absaugen des Katalysators und Entfernen des Lösungsmittels wird der Rückstand in 40 ml absolutem Benzol aufgenommen, mit 0,8 g (3,5 mmol) Dichlordicyanbenzochinon (DDQ) vermischt und 60 Stunden lang gekocht. Nach Absaugen und Einengen der Lösung wird der Rückstand analog Beispiel 3 gereinigt.

Ausbeute: 3,6 g (69 %); farbloses Öl.

Beispiel 9

7-Methoxymethyloxy-2,2-dimethyl-2H-chromen

7,7 g (50 mmol) 3-Methoxymethyloxyphenol werden in Gegenwart von 110 g 50-%iger Schwefelsäure in 150 g Chloroform gelöst und bei 60 °C während 20 Minuten mit 6,8 g Isopren versetzt. Der Ansatz wird 3 Stunden lang bei 60 °C erwärmt, worauf die Phasen getrennt werden. Die organische Phase wird eingeengt und der Rückstand in 100 ml Dioxan in Gegenwart von 2,5 g DDQ 10 Stunden lang gekocht. Im weiteren wird analog Beispiel 8 vorgegangen. Ausbeute: 7,6 g (69 %); hellgelbes Öl.

Beispiel 10

7-Isopropoxy-8-methoxy-2,2-dimethyl-2H-chromen

5,5 g (30 mmol) 2-Methoxy-3-isopropoxyphenol werden in 30 ml Ether gelöst und in kleinen Teilen mit 2,1 g Na vermischt und 1 Stunde lang gekocht. Danach werden 3,2 g (30 mmol) Prenylchlorid zugetropft und 8 Stunden lang gekocht. Die durch übliches Aufarbeiten erhaltenen 4,4 g des Chromanderivats werden in 60 ml Benzol gelöst und in Gegenwart von 1,2 g DDQ 60 Stunden lang gekocht. Das Produkt wird nach üblicher Aufarbeitung analog Beispiel 3 gereinigt. Ausbeute: 3,7 g (50 %); farbloses Öl.

## Beispiel 11

7-s-Butyloxy-8-methoxy-2,2-dimethyl-2H-chromen

Zu einer Lösung von 2 mmol Lithiumdiisopropylamid in 50 ml absolutem Tetrahydrofuran wird bei -30 °C eine Lösung von 2 g (8,4 mmol) 2-Hydroxy-3-methoxy-4-s-butoxyacetophenon in 10 ml Tetrahydrofuran getropft und eine Stunde lang gerührt. Der Ansatz wird auf -40 °C gekühlt, mit 10 mmol Aceton vermischt und zwei Stunden lang gerührt. Danach wird mit Ether verdünnt und angesäuert; die organische Phase wird abgetrennt, das Lösungsmittel entfernt und der Rückstand in 100 ml Methanol gelöst und in Gegenwart von 10 ml conc. Salzsäure 7 Stunden lang gekocht. Anschließend wird auf 200 g Eis gegossen; die Kristalle werden abgesaugt, getrocknet und analog Beispiel 1 aufgearbeitet. Das Produkt wird analog Beispiel 3 gereinigt. Ausbeute: 1,3 g (59 %); farbloses Öl.

In analoger Weise können die in Tabelle 1 aufgeführten Verbindungen hergestellt werden.

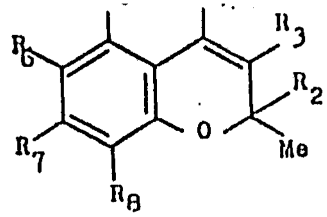

Tabelle 1

| Nr. | $R_2$ | $R_3$ | $R_4$ | $R_5$ | $R_6$ | $R_7$ | $R_8$ | Bsp. Nr. | Ausbeute (%) | NMR |
|---|---|---|---|---|---|---|---|---|---|---|
| 1 | Me | H | H | H | H | nPrO | H | 3 | 72 | 0,94 /3H;t; J=8Hz/; 1,36 /6H;s/; 1,7 /2H;m/; 3,8 /2H;t;J=6Hz/; 5,34 /1H;d;J=10Hz/; 6,16 /1H;d;J=10Hz/; 6,3 /2H;m/; 6,76 /1H;d;J=8Hz/ |
| 2 | Me | H | H | H | H | iPrO | H | 3 | 69 | 1,24 /6H;d;J=6Hz/; 1,36 /6H;s/; 4,4 /1H;m/; 5,34 /1H;d;J=10Hz/ 6,16 /1H;d;J=10Hz/; 6,3 /2H;m/; 6,76 /1H;d;J=8Hz/ |
| 3 | Me | H | H | H | H | iBuO | H | 3 | 64 | 0,96 /6H;d;J=6Hz/; 1,34 /6H;s/; 2,0 /1H;m/; 3,6 /2H;d;J=8Hz/; 5,34 /1H;d;J=10Hz/; 6,16 /1H;d;J=10Hz/; 6,3 /2H;m/; 6,76 /1H;d;J=8Hz/ |
| 4 | Me | H | H | H | H | c-PentylO | H | 3 | 62 | 1,34 /6H;s/; 1,8 /8H;m/; 4,6 /1H;m/; 5,33 /1H;d;J=10Hz/; 6,14 /1H;d;J=10Hz/; 6,28 /2H;m/; 6,72 /1H;d;J=8Hz/; |
| 5 | Me | H | H | H | H | c-HexylO | H | 3 | 59 | 1,34 /6H;s/; 1,6 /10H;m/; 4,56 /1H;m/; 5,34 /1H;d;J=10Hz/; 6,16 /1H;d;J=10Hz/; 6,30 /2H;m/; 6,72 /1H;d;J=8Hz/ |
| 6 | Me | H | H | H | H | $MeOCH_2O$ | H | 9 | 69 | 1,30 /6H;s/; 3,35 /3H;s/; 5,0 /2H;s/; 5,38 /1H;d;J=10Hz/; 6,15 /1H;d;J=10Hz/; 6,45 /2H;m/; 6,75 /1H;d;J=8Hz/ |
| 7 | Me | H | H | H | H | $MeSCH_2O$ | H | 4 | 76 | 1,31 /6H;s/; 2,1 /3H;s/; 5,07 /2H;s/; 5,37 /1H;d;J=10Hz/; 6,13 /1H;d;J=10Hz/; 6,44 /2H;m/; 6,71 /1H;d;J=8Hz/ |
| 8 | Me | H | H | H | H | $EtOCH_2O$ | H | 4 | 69 | 1,2 /3H;t;J=8Hz/; 1,38 /6H;s/; 3,7 /2H;q;J=8Hz/; 5,12 /2H;s/; 5,42 /1H;d;J=10Hz/; 6,2 /1H;d;J=10Hz/; 6,45 /2H;m/; 6,8 /1H;d;J=8Hz/ |
| 9 | Me | H | H | H | H | CrotylO | H | 1 | 76 | 1,45 /6H;s/; 1,77 /3H;d;J=5Hz/; 4,42 /2H;d;J=5Hz/; 5,47 /1H;d;J=10Hz/; 5,77 /2H;m/; 6,27 /1H;d;J=10Hz/; 6,40 /2H;m/; 6,87 /1H;d;J=8Hz/ |
| 10 | Me | H | H | H | H | PrenylO | H | 1 | 82 | 1,45 /6H;s/; 1,77 /6H;m/; 4,45 /2H;d;J=6Hz/; 5,45 /2H;m/; 6,27 /1H;d;J=10Hz/; 6,42 /2H;m/; 6,87 /1H;d;J=8Hz/; |
| 11 | Me | H | H | H | H | BenzylO | H | 5 | 89 | 1,36 /6H;s/; 4,9 /2H;s/; 5,36 /1H;d;J=10Hz/; 6,16 /1H;d;J=10Hz/; 6,4 /2H;m/; 6,76 /1H;d;J=8Hz/; 7,28 /5H;m/ |
| 12 | Me | H | H | H | H | HO-n-ButylO | H | 1 | 72 | 1,4 /6H;s/; 2,85 /4H;m/; 2,45 /1H;sz/; 3,65 /2H;t;J=5Hz/; 3,9 /2H;t;J=5Hz/; 5,45 /1H;d;J=10Hz/; 6,25 /1H;d;J=10Hz/; 6.46 /2H;m/; 6.8 /1H;d;J=8Hz/. |

| | | | | | | | | | | |
|---|---|---|---|---|---|---|---|---|---|---|
| 13 | Me | H | H | H | H | 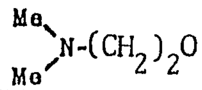 | H | 8 | 57 | 1,41 /6H;s/; 2,30 /6H;s/; 2,7 /2H;t;J=6Hz/; 4,05 /2H;t;J=6Hz/; 5,44 /1H;d;J=10Hz/; 6,22 /1H;d;J=10Hz/; 6,44 /2H;m/; 6,82 /1H;d;J=8Hz/ |
| 14 | Me | H | H | H | H | 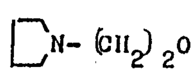 | H | 8 | 61 | 1,42 /6H;s/; 1,78 /4H;m/; 2,59 /4H;m/; 2,90 /2H;t;J=6Hz/; 4,07 /2H;t;J=6Hz/; 5,43 /1H;d;J=10Hz/; 6,21 /1H;d;J=10Hz/; 6,43 /2H;m/; 6,81 /1H;d;J=8Hz/ |
| 15 | Me | H | H | H | H | 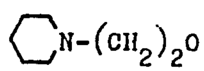 | H | 8 | 49 | 1,43 /6H;s+2H;m/; 1,62 /4H;m/; 2,48 /4H;m/; 2,75 /2H;t;J=6Hz/; 4,05 /2H;t;J=6Hz/; 5,42 /1H;d;J=10Hz/; 6,22 /1H;d;J=10Hz/; 6,44 /2H;m/; 6,80 /1H;d;J=8Hz/ |
| 16 | Me | H | H | H | H | 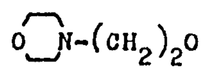 | H | 8 | 63 | 1,42 /6H;s/; 2,55 /4H;m/; 2,75 /2H;t;J=6Hz/; 3,71 /4H;m/; 4,10 /2H;t;J=6Hz/; 5,43 /1H;d;J=10Hz/; 6,22 /1H;d;J=10Hz/; 6,45 /2H;m/; 6,83 /1H;d;J=8Hz/ |
| 17 | Me | H | H | Me | H | EtO | H | 6 | 75 | 1,32 /3H;t;J=6Hz/; 1,36 /6H;s/; 2,2 /3H;s./ 3,9 /2H;q;J=6Hz/; 5,4 /1H;d;J=10Hz/; 6,16 /2H;m/; 6,36 /1H;d;J=10Hz/ |
| 18 | Me | H | H | Me | H | n-PrO | H | 6 | 71 | 0,96 /3H;t;J=6Hz/; 1,36 /6H;s/; 1,7 /2H;m/; 2,16 /3H;s/; 3,8 /2H;t;J=6Hz/; 5,38 /1H;d;J=10Hz/; 6,18 /2H;m/; 6,36 /1H;d;J=10Hz/ |
| 19 | Me | H | H | Me | H | i-PrO | H | 6 | 69 | 1,24 /6H;d;J=6Hz/; 1,36 /6H;s/; 2,16 /3H;s/; 4,4 /1H;m/; 5,38 /1H;d;J=10Hz/; 6,16 /2H;m/; 6,34 /1H;d;J=10Hz/ |
| 20 | Me | H | H | Me | H | s-BuO | H | 6 | 81 | 0,92 /3H;t;J=8Hz/; 1,22 /3H;d;J=4Hz/; 1,34 /6H;s/; 1,6 /2H;m/; 2,18 /3H;s/; 4,2 /1H;m/; 5,4 /1H;d;J=10Hz/; 6,16 /2H;m/; 6,36 /1H;d;J=10Hz/ |
| 21 | Me | H | H | Me | H | i-BuO | H | 6 | 73 | 0,96 /6H;d;J=7Hz/; 1,35 /6H;s/; 2,0 /1H;s/; 2,18 /3H;s/; 3,6 /2H;d;J=7Hz/; 5,4 /1H;d;J=10Hz/; 6,2 /2H;m/; 6,36 /1H;d;J=10Hz/ |
| 22 | Me | H | H | Me | H | c-PentylO | H | 3 | 74 | 1,36 /6H;s/; 1,8 /8H;m/; 2,16 /3H;s/; 4,6 /1H;m/; 5,36 /1H;d;J=10Hz/; 6,16 /2H;m/; 6,36 /1H;d;J=10Hz/ |
| 23 | Me | H | H | Me | H | c-HexylO | H | 9 | 69 | 1,40 /6H;s/; 1,2-2,05 /10H;m/; 2,25 /3H;s/; 4,1 /1H;m/; 5,5 /1H;d;J=10Hz/; 6,25 /2H;m/; 6,42 /1H;d;J=10Hz/ |
| 24 | Me | H | H | Me | H | $MeOCH_2O$ | H | 10 | 46 | 1,42 /6H;s/; 2,25 /3H;s/; 3,45 /3H;s/; 5,1 /2H;s/; 5,51 /1H;d;J=10Hz/; 6,37 /2H;m/; 6,45 /1H;d;J=10Hz/ |

| | | | | | | | | | |
|---|---|---|---|---|---|---|---|---|---|
| 25 Me | H | H | Me | H | $MeSCH_2O$ | H | 10 | 49 | 1,44 /6H;s/; 2,09 /3H,s/; 2,24 /3H;s/; 5,04 /2H;s/; 5,43 /1H;d;J=10Hz/; 6,29 /2H;m/; 6,39 /1H;d;J=.8Hz/ |
| 26 Me | H | H | Me | H | $EtOCH_2O$ | H | 10 | 53 | 1,21 /3H;t;J=8Hz/; 1,43 /6H;s/; 2,23 /3H;s/; 3,69 /2H;q; J=8Hz/; 5,10 /2H;s/; 5,44 /1H;d;J=10Hz/; 6,27 /2H;m/; 6,41 /1H;d;J=10 Hz/ |
| 27 Me | H | H | Me | H | AllylO | H | 1 | 76 | 1,45 /6H;s/; 2,25 /3H;s/; 4,47 /2H;m/; 5,32 /2H;m/; 5,52 /1H; d;J=10Hz/; 6,05 /1H;m/; 6,27 /2H;m/; 6,45 /1H;d;J=10Hz/; |
| 28 Me | H | H | Me | H | CrotylO | H | 1 | 82 | 1,43 /6H;s/; 1,75 /3H;m/; 2,25 /3H;s/; 4,4 /2H;m/; 5,5 /1H;d; J=10Hz/; 5,77 /2H;m/; 6,27 /2H;m/; 6,43 /1H;d;J=10Hz/ |
| 29 Me | H | H | Me | H | PrenylO | H | 1 | 87 | 1,43 /6H;s/; 1,77 /6H;m/; 2,27 /3H;s/; 4,45 /2H;m/; 5,5 /2H;m/; 6,28 /2H;m/; 6,45 /1H;d;J=10Hz/ |
| 30 Me | H | H | Me | H | $CH{\equiv}C{-}CH_2O$ | H | 1 | 79 | 1,36 /6H;s/; 2,2 /3H;s/; 2,44 /1H;t;J=2Hz/; 4,54 /2H;d;J=2Hz/; 5,44 /1H;d;J=10Hz/; 6,24 /2H;m/; 6,36 /1H;d;J=10Hz/ |
| 31 Me | H | H | Me | H | BenzylO | H | 7 | 47 | 1,42 /6H;s/; 2,25 /3H;s/; 4,97 /2H;s/; 5,49 /1H;d;J=10Hz/; 6,35 /2H;m/; 7,35 /5H;m/ |
| 32 Me | H | H | Me | H | $Me_2N{-}(CH_2)_2O$ | H | 8 | 61 | 1,42 /6H;s/; 2,23 /3H;s/; 2,3 /6H;s/; 2,7 /2H;t;J=6Hz/; 4,03 /2H;t;J=6Hz/; 5,48 /1H;d;J=10Hz/; 6,26 /2H;m/; 6,42 /1H;d; J=10Hz/ |
| 33 Me | H | H | Me | H | 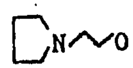 | H | 8 | 48 | 1,43 /6H;s/; 1,78 /4H;m/; 2,24 /3H;s/; 2,59 /4H;m/; 2,91 /2H;t;J=6Hz/; 4,06 /2H;t;J=6Hz/; 5,47 /1H;d;J=10Hz/; 6,24 /2H;m/; 6,43 /1H;d;J=10Hz/ |
| 34 Me | H | H | Me | H | 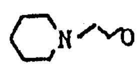 | H | 8 | 57 | 1,42 /6H;s/;+2H;m/; 1,60 /4H;m/; 2,25 /3H;s/; 2,49 /4H;m/; 2,75 /2H;t;J=6Hz/; 4,04 /2H;t;J=6Hz/; 5,46 /1H;d;J=10Hz/; 6,26 /2H;m/; 6,41 /1H;d;J=10Hz/ |
| 35 Me | H | H | Me | H | 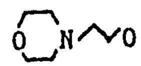 | H | 8 | 62 | 1,42 /6H;s/; 2,25 /3H;s/; 2,56 /4H;m/; 2,72 /2H;t;J=6Hz/; 3,70 /4H;m/; 4,08 /2H;t;J=6Hz/; 5,47 /1H;d;J=10Hz/; 6,25 /2H;m/; 6,42 /1H;d;J=10Hz/ |
| 36 Me | H | H | H | H | EtO | Me | 7 | 50 | 1,42 /6H;s;+3H;t;J=7Hz/; 2,1 /3H;s/; 4,0 /2H;q;J=7Hz/; 5,47 /1H;d;J=10Hz/; 6,27 /1H;d;J=10Hz/; 6,35 /1H;d;J=8Hz/; 6,75 /1H;d;J=8Hz/ |
| 37 Me | H | H | H | H | n-PrO | Me | 4 | 69 | 1,05 /3H;t;J=8Hz/; 1,42 /6H;s/; 1,57 /2H;m/; 2,1 /3H;s/; 3,9 /2H;t;J=6Hz/; 5,47 /1H;d;J=10Hz/; 6,25 /1H;d;J=10Hz/; 6,35 /1H;d;J=8Hz/; 6,75 /1H;d;J=8Hz/ |

| | | | | | | | | | | |
|---|---|---|---|---|---|---|---|---|---|---|
| 38 | Me | H | H | H | H | i-PrO | Me | 4 | 78 | 1,35 /6H;d;J=6Hz/; 1,42/6H;s/; 2,1 /3H;s/; 4,47 /1H;m/; 5,45 /1H;d;J=10Hz/; 6,25 /1H;d;J=10Hz/; 6,35 /1H;d;J=8Hz/; 6,75 /1H;d;J=8Hz/ |
| 39 | Me | H | H | H | H | s-BuO | Me | 4 | 82 | 0,97 /3H;t;J=8Hz/; 1,3 /3H;d;J=5Hz/; 1,42 /6H;s/; 1,68 /2H;m/; 2,1 /3H;s/; 4,25 /1H;m/; 5,45 /1H;d;J=10Hz/; 6,25 /1H;d;J=10Hz/ 6,35 /1H;d;J=8Hz/; 6,72 /1H;d;J=8Hz/ |
| 40 | Me | H | H | H | H | i-BuO | Me | 4 | 80 | 1,05 /6H;d;J=7Hz/; 1,46 /6H;s/; 2,1 /4H;s+m/; 3,7 /2H;d;J=8Hz/; 5,47 /1H;d;J=10Hz/; 6,27 /1H;d;J=10Hz/; 6,35 /1H;d;J=8Hz/; 6,75 /1H;d;J=8Hz/ |
| 41 | Me | H | H | H | H | c-PentylO | Me | 5 | 89 | 1,43 /6H;s/; 1,6 /2H;m/; 1,85 /6H;m/; 2,07 /3H;s/; 4,72 /1H;m/; 5,45 /1H;d;J=10Hz/; 6,25 /1H;d;J=10Hz/; 6,35 /1H;d;J=8Hz/; 6,72 /1H;d;J=8Hz/ |
| 42 | Me | H | H | H | H | c-HexylO | Me | 5 | 79 | 1,43 /6H;s/; 1,2-2,0 /10H;m/; 2,05 /3H;s/; 4,18 /1H;m/; 5,44 /1H;d;J=10Hz/; 6,25 /1H;d;J=10Hz/; 6,36 /1H;d;J=8Hz/; 6,73 /1H;d;J=8Hz/ |
| 43 | Me | H | H | H | H | $MeOCH_2O-$ | Me | 9 | 66 | 1,45 /6H;s/; 2,1 /3H;s/; 3,5 /3H;s/; 5,17 /2H;s/; 5,5 /1H;d; J=10Hz/; 6,27 /1H;d;J=10Hz/; 6,57 /1H;d;J=8Hz/; 6,77 /1H;d; J=8Hz/ |
| 44 | Me | H | H | H | H | $MeS-CH_2O-$ | Me | 9 | 61 | 1,42 /6H;s/; 2,06 /3H;s/; 2,09 /3H;s/; 5,05 /2H;s/; 5,5 /1H; d;J=10Hz/; 6,26 /1H;d;J=10Hz/; 6,56 /1H;d;J=8Hz/; 6,76 /1H;d; J=8Hz/ |
| 45 | Me | H | H | H | H | $EtOCH_2O$ | Me | 4 | 76 | 1,20 /3H;t;J=8Hz/; 1,42 /6H;s/; 2,05 /3H;s/; 3,68 /2H;q;J=8Hz/; 5,12 /2H;s/; 5,49 /1H;d;J=10Hz/; 6,25 /1H;d;J=10Hz/; 6,55 /1H;d;J=8Hz/; 6,75 /1H;d;J=8Hz/ |
| 46 | Me | H | H | H | H | AllylO | Me | 1 | 82 | 1,42 /6H;s/; 2,1 /3H;s/; 4,5 /2H;m/; 5,32 /2H;m/; 5,5 /1H;d; J=10Hz/; 6,05 /1H;m/; 6,25 /1H;d;J=10Hz/; 6,35 /1H;d;J=8Hz/; 6,75 /1H;d;J=8Hz/ |
| 47 | Me | H | H | H | H | CrotylO | Me | 1 | 76 | 1,42 /6H;s/; 1,77 /3H;m/; 2,1 /3H;s/; 4,42 /2H;m/; 5,45 /1H; d;J=10Hz/; 5,75 /2H;m/; 6,25 /1H;d;J=10Hz/; 6,35 /1H;d;J=8Hz/; 6,75 /1H;d;J=8Hz/ |
| 48 | Me | H | H | H | H | PrentylO | Me | 1 | 69 | 1,42 /6H;s/; 1,75 /6H;m/; 4,5 /2H;m/; 5,47 /2H;m/; 6,25 /1H; d;J=10Hz/; 6,37 /1H;d;J=8Hz/; 6,75 /1H;d;J=8Hz/ |
| 49 | Me | H | H | H | H | BenzylO | Me | 6 | 78 | 1,45 /6H;s/; 2,1 /3H;s/; 5,05 /2H;s/; 5,47 /1H;d;J=10Hz/; 6,25 /1H;d;J=10Hz/; 6,4 /1H;d;J=8Hz/; 6,75 /1H;d;J=8Hz/; 7,37 /5H;m/ |
| 50 | Me | H | H | H | H | $CH\equiv C-CH_2O$ | Me | 1 | 81 | 1,42 /6H;s/; 2,1 /3H;s/; 2,46 /2H;t;J=2Hz/; 5,44 /1H;d;J=2Hz/; 5,45 /1H;d;J=10Hz/; 6,24 /1H;d;J=10Hz/; 6,4 /1H;d;J=8Hz/; 6,75 /1H;d;J=8Hz/ |

| | | | | | | | | | |
|---|---|---|---|---|---|---|---|---|---|
| 51 Me | H | H | H | H | Me, Me N-$(CH_2)_2O$ | Me | 8 | 63 | 1,42 /6H;s/; 2,05 /3H;s/; 2,3 /6H;s/; 2,69 /2H;... 4,02 /2H;t;J=6Hz/; 5,48 /1H;d;J=10Hz/; 6,24 /1H;d;J=10Hz/; 6,54 /1H;d;J=8Hz/; 6,75 /1H;d;J=8Hz/ |
| 52 Me | H | H | H | H | 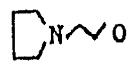 | Me | 8 | 59 | 1,42 /6H;s/; 1,78 /4H;m/; 2,04 /3H;s/; 2,59 /4H;m/; 2,90 /2H;t;J=6Hz/; 4,05 /2H;t;J=6Hz/; 5,47 /1H;d;J=10Hz/; 6,23 /1H;d;J=10Hz/; 6,55 /1H;d;J=8Hz/; 6,74 /1H;d;J=8Hz/ |
| 53 Me | H | H | H | H | 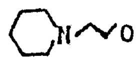 | Me | 8 | 65 | 1,42 /6H;s+2H;m/; 1,6 /4H;m/; 2,05 /3H;s/; 2,49 /4H;m/; 2,76 /2H;t;J=6Hz/; 4,07 /2H;t;J=6Hz/; 5,48 /1H;d;J=10Hz/; 6,22 /1H;d;J=10Hz/; 6,53 /1H;d;J=8Hz/; 6,72 /1H;d;J=8Hz/ |
| 54 Me | H | H | H | H | 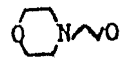 | Me | 8 | 70 | 1,42 /6H;s/; 2,05 /3H;s/; 2,55 /4H;m/; 2,71 /2H;t;J=6Hz/; 3,71 /4H;m/; 4,1 /2H;t;J=6Hz/; 5,47 /1H;d;J=10Hz/; 6,23 /1H;d;J=10Hz 6,54 /1H;d;J=8Hz/; 6,73 /1H;d;J=8Hz/ |
| 55 Me | H | H | H | i-BuO | i-BuO | H | 11 | 47 | 0,96 /12H;d;J=8Hz/; 1,32 /6H;s/; 2,0 /2H;m/; 3,62 /4H;d;J=6Hz/; 5,34 /1H;d;J=10Hz/; 6,12 /1H;d;J=10Hz/; 6,32 /1H;s/; 6,47 /1H;s/ |
| 56 Me | H | H | H | Benzyl0 | Benzyl0 | H | 1 | 81 | 1,32 /6H;s/; 4,93 /2H;s/; 4,96 /2H;s/; 5,34 /1H;d;J=10Hz/; 6,12 /1H;d;J=10Hz/; 6,41 /1H;s/; 6,54 /11H;m/ |
| 57 Me | H | H | H | $CF_3CH_2O$ | $CF_3CH_2O$ | H | 1 | 61 | 1,4 /6H;s/; 4,2 /4H;m/; 5,54 /1H;d;J=10Hz/; 6,2 /1H;d;J=10Hz/; 6,42 /1H;s/; 6,66 /1H;s/ |
| 58 Me | H | H | H | Allyl0 | Allyl0 | H | 1 | 82 | 1,36 /6H;s/; 4,5 /4H;m/; 5,3 /5H;m/; 6,0/3H;m/; 6,36 /1H;s/; 6,52 /1H;s/ |
| 59 Me | H | H | H | Crotyl0 | Crotyl0 | H | 1 | 69 | 1,4 /6H;s/; 1,77 /6H;m/; 4,5 /4H;m/; 5,58 /1H;d;J=10Hz/; 5,9 /4H;m/; 6,36 /1H;d;J=10Hz/; 6,58 /1H;s/; 6,72 /1H;s/ |
| 60 Me | H | H | H | Prenyl0 | Prenyl0 | H | 1 | 72 | 1,4 /6H;s/; 1,75 /12H;m/; 4,6 /4H;m/; 5,6 /3H;m/; 6,32 /1H; d;J=10Hz/; 6,52 /1H;s/; 6,62 /1H;s/ |
| 61 Me | H | H | H | CH≡C-$CH_2O$ | CH≡C-$CH_2O$ | H | 1 | 80 | 1,35 /6H;s/; 2,45 /2H;m/; 4,57 /2H;m/; 4,61 /2H;m/; 5,4 /1H; d;J=10Hz/; 6,12 /1H;d;J=10Hz/; 6,42 /1H;s/; 6,62 /1H;s/ |
| 62 Me | Cl | Cl | H | EtO | EtO | H | 2 | 58 | 1,35 /6H;m/; 1,48 /6H;s/; 4,1 /4H;m/; 6,38 /1H;s/; 6,92 /1H;s/ |
| 63 Me | Cl | Cl | H | i-PrO | i-PrO | H | 2 | 62 | 1,3 /12H;m/; 1,48 /6H;s/; 4,4 /2H;m/; 6,4 /1H;s/; 7,0 /1H;s/ |
| 64 Me | Cl | Cl | H | MeO | EtO | H | 2 | 57 | 1,48 /3H;t;J=5Hz/; 1,6 /6H;s/; 3,8 /3H;s/; 4,1 /2H;q;J=5Hz/; 6,36 /1H;s/; 6,88 /1H;s/ |
| 65 Me | Cl | Cl | H | MeO | i-PrO | H | 2 | 60 | 1,32 /6H;d;J=8Hz/; 1,48 /6H;s/; 3,76 /3H;s/; 4,45 /1H;m/; 6,4 /1H;s/; 6,88 /1H;s/ |
| 66 Me | H | H | H | MeO | s-BuO | H | 5 | 76 | 0,92 /3H;t;J=8Hz/; 1,23 /3H;d;J=6Hz/; 1,32 /6H;s/; 1,64 /2H;m/; 3,66 /3H;s/; 4,15 /1H;m/; 5,36 /1H;d;J=10Hz/; 6,14 /1H;d; J=10Hz/; 6,32 /1H;s/; 6,44 /1H;s/ |

| | | | | | | | | | |
|---|---|---|---|---|---|---|---|---|---|
| 67 Me | H | H | H | MeO | i-BuO | H | 5 | 84 | 0,98 /6H;d;J=8Hz/; 1,32 /6H;s/; 2,0 /1H;m/; 3,64 /2H;d;J=6Hz/; 3,68 /3H;s/; 5,36 /1H;d;J=10Hz/; 6,14 /1H;d;J=10Hz/; 6,32 /1H;s/; 6,44 /1H;s/ |
| 68 Me | H | H | H | MeO | c-Pentyl0 | H | 5 | 79 | 1,42 /6H;s/; 1,6 /2H;m/; 1,92 /6H;m/; 3,8 /3H;s/; 4,72 /1H;m/; 5,47 /1H;d;J=10Hz/; 6,25 /1H;d;J=10Hz/; 6,4 /1H;s/; 6,52 /1H;s/ |
| 69 Me | H | H | H | MeO | c-Hexyl0 | H | 5 | 83 | 1,40 /6H;s/; 1,19-2,0 /10H;m/; 3,81 /3H;s/; 4,12 /1H;m/; 5,46 /1H;d;J=10Hz/; 6,23 /1H;d;J=10Hz/; 6,41 /1H;s/; 6,53 /1H;s/ |
| 70 Me | H | H | H | MeO | Benzyl0 | H | 5 | 90 | 1,32 /6H;s/; 3,69 /3H;s/; 4,99 /2H;s/; 5,36 /1H;d;J=10Hz/; 6,16 /1H;d;J=10Hz/; 6,4 /1H;s/; 6,48 /1H;s/; 7,3 /5H;m/ |
| 71 Me | H | H | H | | O-$(CH_3)_2$C-O | H | 11 | 47 | 1,4 /6H;s/; 1,65 /6H;s/; 5,45 /1H;d;J=10Hz/; 6,2 /1H;d;J=10Hz/; 6,3 /1H;s/; 6,4 /1H;s/ |
| 72 Me | H | H | H | MeO | Crotyl0 | H | 1 | 69 | 1,40 /6H;s/; 1,70 /3H;m/; 3,84 /3H;s/; 4,59 /2H;m/; 5,36 /1H;d;J=10Hz/; 5,9 /2H;m/; 6,17 /1H;d;J=10Hz/; 6,3 /1H;s/; 6,42 /1H;s/ |
| 73 Me | H | H | H | MeO | Prenyl0 | H | 1 | 74 | 1,4 /6H;s/; 1,75 /6H;m/; 3,8 /3H;s/; 4,66 /2H;m/; 5,6 /2H;m/; 6,36 /1H;d;J=10Hz/; 6,58 /1H;s/; 6,68 /1H;s/ |
| 74 Me | H | H | H | | O-$(CH_2)_3$-O | H | 10 | 47 | 1,42 /6H;s/; 2,27 /2H;m/; 4,07 /4H;m/; 5,47 /1H;d;J=10Hz/; 6,20 /1H;d;J=10Hz/; 6,4 /1H;s/; 6,57 /1H;s/ |
| 75 Me | H | H | H | MeO | $MeOCH_2O$ | H | 4 | 76 | 1,42 /6H;s/; 3,34 /3H;s/; 3,81 /3H;s/; 5,01 /2H;s/; 5,48 /1H;d;J=10Hz/; 6,21 /1H;d;J=10Hz/; 6,41 /1H;s/; 6,58 /1H;s/ |
| 76 Me | H | H | H | MeO | $MeSCH_2O$ | H | 4 | 59 | 1,42 /6H;s/; 2,1 /3H;s/; 3,8 /3H;s/; 5,08 /2H;s/; 5,46 /1H;d;J=10Hz/; 6,20 /1H;d;J=10Hz/; 6,41 /1H;s/; 6,56 /1H;s/ |
| 77 Me | H | H | H | H | EtO | EtO | 7 | 46 | 1,30 /3H;t;J=6Hz/; 1,41 /3H;t;J=6Hz/; 1,45 /6H;s/; 4,03 /4H;m/; 5,51 /1H;d;J=10Hz/; 6,28 /1H;d;J=10Hz/; 6,39 /1H;d;J=8Hz/; 6,62 /1H;d;J=8Hz/ |
| 78 Me | H | H | H | H | n-PrO | n-PrO | 7 | 49 | 1,07 /6H;m/; 1,42 /6H;s/; 1,5-2,0 /4H;m/; 3,9-4,1 /4H;m/; 5,52 /1H;d;J=10Hz/; 6,27 /1H;d;J=10Hz/; 6,30 /1H;d;J=8Hz/; 6,61 /1H;d;J=8Hz/ |

| | | | | | | | | | | |
|---|---|---|---|---|---|---|---|---|---|---|
| 79 | Me | H | H | H | H | i-PrO | i-PrO | 7 | 52 | 1,30 /12H;m/; 1,4 /6H;s/; 4,20 /1H;m/; 4,5 /1H;m/; 5,50 /1H;d;J=10Hz/; 6,26 /1H;d;J=10Hz/; 6,36 /1H;d;J=8Hz/; 6,60 /1H;d;J=8Hz/ |
| 80 | Me | H | H | H | H | s-BuO | s-BuO | 7 | 51 | 1,02 /6H;m/; 1,2 /6H;m/; 1,45 /6H;s/; 1,5-2,0 /4H;m/; 4,1 /1H;m/; 4,36 /1H;m/; 5,52 /1H;d;J=10Hz/; 6,28 /1H;d;J=10Hz/; 6,37 /1H;d;J=8Hz/; 6,61 /1H;d;J=8Hz/ |
| 81 | Me | H | H | H | H | i-BuO | i-BuO | 7 | 49 | 1,05 /12H;m/; 1,42 /6H;s/; 1,9-2,1 /2H;m/; 3,6-3,77 /4H;m/; 5,52 /1H;d;J=10Hz/; 6,26 /1H;d;J=10Hz/; 6,36 /1H;d;J=8Hz/; 6,60 /1H;d;J=8Hz/ |
| 82 | Me | H | H | H | H | $O-CH_2-O$ | | 9 | 63 | 1,47 /6H;s/; 5,5 /1H;d;J=10Hz/; 5,95 /2H;s/; 6,27 /1H;d;J=10Hz/; 6,38 /1H;d;J=8Hz/; 6,47 /1H;d;J=8Hz/; |
| 83 | Me | H | H | H | H | $O-(CH_2)_2-O$ | | 9 | 61 | 1,47 /6H;s/; 4,25 /4H;m/; 5,47 /1H;d;J=10Hz/; 6,25 /1H;d;J=10Hz/; 6,37 /1H;d;J=8Hz/; 6,5 /1H;d;J=8Hz/ |
| 84 | Me | H | H | H | H | $O-(CH_2)_3-O$ | | 9 | 69 | 1,47 /6H;s/; 2,17 /2H;m/; 4,25 /4H;m/; 5,5 /1H;d;J=10Hz/; 6,25 /1H;d;J=10Hz/; 6,45 /1H;d;J=8Hz/; 6,55 /1H;d;J=8Hz/ |
| 85 | Me | H | H | H | H | $O-(CH_2)_2-O-(CH_2)_2-O$ | | 9 | 48 | 1,45 /6H;s/; 3,52 /2H;m/; 3,75 /4H;m/; 4,05 /2H;m/; 5,5 /1H;d;J=10Hz/; 6,22 /1H;d;J=10Hz/; 6,38 /1H;d;J=8Hz/; 6,6 /1H;d;J=8Hz/ |
| 86 | Me | H | H | H | H | BenzylO | BenzylO | 11 | 53 | 1,42 /6H;s/; 4,91 /2H;s/; 4,97 /2H;s/; 5,50 /1H;d;J=10Hz/; 6,22 /1H;d;J=10Hz/; 6,38 /1H;d;J=8Hz/; 6,5-6,6 /11H;m/ |
| 87 | Me | H | H | H | H | EtO | MeO | 11 | 49 | 1,38 /3H;t;J=8Hz/; 1,42 /6H;s/; 3,8 /3H;s/; 4,0 /2H;q;J=8Hz/; 5,44 /1H;d;J=10Hz/; 6,2 /1H;d;J=10Hz/; 6,32 /1H;d;J=8Hz/; 6,56 /1H;d;J=8Hz/ |
| 88 | Me | H | H | H | H | n-PrO | MeO | 6 | 80 | 1,02 /3H;t;J=7Hz/; 1,40 /6H;s/; 1,8 /2H;m/; 3,8 /3H;s/; 3,83 /2H;q;J=7Hz/; 5,42 /1H;d;J=10Hz/; 6,19 /1H;d;J=10Hz/; 6,32 /1H;d;J=8Hz/; 6,56 /1H;d;J=8Hz/ |
| 89 | Me | H | H | H | H | s-BuO | MeO | 11 | 59 | 0,96 /3H;t;J=8Hz/; 1,24 /3H;d;J=7Hz/; 1,41 /6H;s/; 1,7 /2H;m/; 3,76 /3H;s/; 4,2 /1H;m/; 5,4 /1H;d;J=10Hz/; 6,18 /1H;d;J=10Hz/; 6,32 /1H;d;J=8Hz/; 6,56 /1H;d;J=8Hz/ |
| 90 | Me | H | H | H | H | i-PrO | MeO | 10 | 50 | 1,28 /6H;d;J=7Hz/; 1,42 /6H;s/; 3,76 /3H;s/; 4,44 /1H;m/; 5,4 /1H;d;J=10Hz/; 6,16 /1H;d;J=10Hz/; 6,32 /1H;d;J=8Hz/; 6,56 /1H;d;J=8Hz/ |
| 91 | Me | H | H | H | H | i-BuO | MeO | 4 | 80 | 1,0 /6H;d;J=8Hz/; 1,4 /6H;s/; 2,0 /1H;m/; 3,66 /2H;d;J=8Hz/; 3,78 /3H;s/; 5,4 /1H;d;J=10Hz/; 6,16 /1H;d;J=10Hz/; 6,32 /1H;d;J=8Hz/; 6,56 /1H;d;J=8Hz/ |
| 92 | Me | H | H | H | H | c-PentylO | MeO | 10 | 52 | 1,42 /6H;s/; 1,6 /2H;m/; 1,90 /6H;m/; 3,8 /3H;s/; 4,70 /1H;m/; 5,45 /1H;d;J=10Hz/; 6,22 /1H;d;J=10Hz/; 6,34 /1H;d;J=8Hz/; 6,58 /1H;d;J=8Hz/ |

0208

| | | | | | | | | | | |
|---|---|---|---|---|---|---|---|---|---|---|
| 93 | Me | Cl | Cl | H | H | MeO | MeO | 2 | 57 | 1,6 /6H;s/; 3,82 /3H;s/; 3,84 /3H;s/; 6,51 /1H;d;J=8Hz/; 7,09 /1H;d;J=8Hz/ |
| 94 | Me | Cl | Cl | H | H | EtO | MeO | 2 | 61 | 1,45 /3H;t;J=6Hz/; 1,6 /6H;s/; 3,85 /3H;s/; 4,1 /2H;q; J=6Hz/; 6,52 /1H;d;J=8Hz/; 7,1 /1H;d;J=8Hz/ |
| 95 | Me | Cl | Cl | H | H | i-PrO | MeO | 2 | 49 | 1,37 /6H;d;J=6Hz/; 1,6 /6H;s/; 3,82 /3H;s/; 4,65 /1H;m/; 6,55 /1H;d;J=8Hz/; 7,1 /1H;d;J=8Hz/ |
| 96 | Me | Cl | Cl | H | H | s-BuO | MeO | 2 | 63 | 0,93 /3H;t;J=8Hz/; 1,24 /3H;d;J=6Hz/; 1,61 /6H;s/; 1,65 /2H;m/; 3,79 /3H;s/; 4,16 /1H;m/; 6,54 /1H;d;J=8Hz/; 7,11 /1H;d;J=8Hz/ |
| 97 | Me | Cl | Cl | H | H | i-BuO | MeO | 2 | 56 | 0,98 /6H;d;J=8Hz/; 1,60 /6H;s/; 2,0 /1H;m/; 3,69 /2H;d; J=6Hz/; 3,81 /3H;s/; 6,56 /1H;d;J=8Hz/; 7,12 /1H;d; J=8Hz/ |
| 98 | Me | Cl | Cl | H | H | c-Pentyl0 | MeO | 2 | 51 | 1,6 /6H;s+2H;m/; 1,7-2,0 /6H;m/; 3,81 /3H;s/; 4,75 /1H;m/; 6,55 /1H;d;J=8Hz/; 7,11 /1H;d;J=8Hz/ |
| 99 | Me | H | H | MeO | H | EtO | H | 8 | 57 | 1,32 /3H;t;J=7Hz/; 1,36 /6H;s/; 3,68 /3H;s/; 3,9 /2H;q; J=7Hz/; 5,3 /1H;d;J=10Hz/; 5,92 /2H;m/; 6,51 /1H;d;J=10Hz/ |
| 100 | Me | H | H | MeO | H | n-PrO | H | 5 | 86 | 0,98 /3H;t;J=6Hz/; 1,35 /6H;s/; 1,7 /2H;m/; 3,7 /3H;s/; 3,8 /2H;t;J=7Hz/; 5,32 /1H;d;J=10Hz/; 5,93 /2H;m/; 6,52 /1H; d;J=10Hz/ |
| 101 | Me | H | H | MeO | H | i-PrO | H | 5 | 79 | 1,20 /6H;d;J=8Hz/; 1,36 /6H;s/; 3,7 /3H;s/; 4,44 /1H;m/; 5,32 /1H;d;J=10Hz/; 5,92 /2H;m/; 6,52 /1H;d;J=10Hz/ |
| 102 | Me | H | H | MeO | H | s-BuO | H | 5 | 95 | 0,93 /3H;t;J=7Hz/; 1,24 /3H;d;J=7Hz/; 1,36 /6H;s/; 1,6 /2H; m/; 3,7 /3H;s/; 4,2 /1H;m/; 5,31 /1H;d;J=10Hz/; 5,92 /2H;m/; 6,51 /1H;d;J=10Hz/ |
| 103 | Me | H | H | MeO | H | i-BuO | H | 8 | 69 | 0,96 /6H;d;J=6Hz/; 1,36 /6H;s/; 2,0 /1H;m/; 3,62 /2H;d; J=6Hz/; 3,7 /3H;s/; 5,32 /1H;d;J=10Hz/; 5,94 /2H;m/; 6,5 /1H;d;J=10Hz/ |
| 104 | Me | H | H | MeO | H | c-Pentyl0 | H | 11 | 53 | 1,42 /6H;s/; 1,6 /2H;m/; 1,92 /6H;m/; 3,8 /3H;s/; 4,71 /1H;m/; 5,32 /1H;d;J=10Hz/; 5,93 /2H;m/; 6,52 /1H;d;J=10Hz/ |

Beispiel 105

7-(o-Chlorbenzyloxy)-2,2-dimethyl-2H-chromen

Hergestellt gemäß Beispiel 1.

Ausbeute: 65 %; Öl.

NMR: 1,42 (6H;s); 5,1 (2H;s); 5,47 (1H;d;J=10Hz); 6,27 (1H;d;J=10Hz); 6,5 (2H;m); 6,9 (1H;d; J=8Hz); 7,25 (2H;m); 7,31 (1H;m); 7,31 (1H;m); 7,50 (1H;m).

Beispiel 106

7-(p-Chlorbenzyloxy)-2,2-dimethyl-2H-chromen

Hergestellt gemäß Beispiel 1.

Ausbeute: 74 %. Fp.: 98 - 100 °C.

NMR: 1,42 (6H;s); 4,97 (2H;s); 5,47 (1H;d;J=10Hz); 6,37 (1H;d;J=10Hz); 6,45 (2H;m); 6,77 (1H;d; J=10Hz); 7,37 (4H;s).

Beispiel 107

3,4-Dichlor-7-propargyloxy-2,2-dimethyl-2H-chromen

Hergestellt gemäß Beispiel 2.

Ausbeute: 81 %. Fp.: 479 °C.

NMR: 1,55 (6H;s); 2,58 (1H;t;J=2Hz); 4,65 (2H;d; J=2Hz); 6,46 (1H;d;J=2Hz); 6,55 (1H;dd; $J_1$=2Hz;$J_2$=8Hz); 7,26 (1H;d; J=8Hz).

Beispiel 108

Herstellung eines Emulsionspräparates

Die folgenden Komponenten werden miteinander vermischt, dann bei Raumtemperatur 1 Stunde lang gerührt; 50 Massenteile Verbindung Nr. 21, 40 Massenteile Xylol, 10 Massenteile Dodecylbenzolsulfonsäure-Na. Die Zusammensetzung wird mit Wasser auf die notwendige Wirkstoffkonzentration verdünnt und als Spritzmittel verwendet.

Beispiel 109

Herstellung eines Suspensionspräparates

Die folgenden Komponenten werden miteinander vermischt, dann 2 Stunden lang bei Raumtemperatur homogenisiert: 40 Massenteile Verbindung Nr. 107, 15 Massenteile Diatomeenerde, 15 Massenteile Ton, 15 Massenteile Knochenkohle, 5 Massenteile Polyglycolphenylether. Anwendung als Spritzmittel.

Beispiel 110

Herstellung eines Pulverpräparates

Die folgenden Komponenten werden unter Standardbedingungen gemahlen: 10 Massenteile Verbindung Nr. 88, 47 Massenteile Talkum, 47 Massenteile Ton, 3 Massenteile Knochenkohle. Das Präparat wird pulverisiert und zum Einstäuben verwendet.

Beispiel 111

Herstellung eines Granulates

Die folgenden Komponenten werden miteinander vermischt; 5 Massenteile Verbindung Nr. 102, 15 Massenteile Bentonit, 47,5 Massenteile Talkum, 30 Massenteile Ton, 2 Massenteile Dodecylbenzolsulfon-

säure-Na, 25 Massenteile Wasser. Das Gemisch wird in einer Granuliermaschine granuliert; das Granulat wird getrocknet und zur Anwendung in den Boden eingebracht.

Beispiel 112

Herstellung eines Emulsionspräparates

Die folgenden Komponenten werden miteinander vermischt: 25 Massenteile Verbindung Nr. 30, 30 Massenteile Xylol, 30 Massenteile Dimethylsulfoxid, 15 Massenteile Dodecylbenzolsulfonsäure-Na. Es wird 1 Stunde lang bei Raumtemperatur gerührt. Vor der Anwendung wird mit Wasser auf die gewünschte Wirkstoffkonzentration verdünnt und dann gespritzt.

Beispiel 113

Herstellung eines Pulverpräparates

Die folgenden Komponenten werden miteinander vermischt: 0,5 Massenteile Verbindung Nr. 102, 99,5 Massenteile Talkum. Es wird unter Standardbedingungen gemahlen, dann pulverisiert und zur Anwendung ausgestreut.

Untersuchung der insektenwachstumshemmenden und nematociden Wirkung der Verbindungen der Tabelle 1:

Testtiere:

Insekten: Baumwollwanze (Dysdercus fasciatus)
Kohlraupe (Pieris brassicae)
Senfblattkäfer (Colaphellus sophiae)
Stubenfliege (Musca domestica)
Erbsenblattlaus (Acyrthosyphon pisum)
Kartoffelkäfer (Leptinotarsa decemlineata)

Fadenwürmer: Caenorhabditis elegans
Wurzelgallennematode (Meloidogyne marioni)

Die einzelnen Versuche an den Testtieren wurden wie folgt durchgeführt.

1) Baumwollwanze:

Es wurde aus den Wirkstoffen eine Reihe von Lösungen in Aceton mit bestimmter Konzentrationsabstufung hergestellt, von denen je 0,2 µl auf die Rückenseite von jeweils 50 Larven, die sich im II. Stadium befanden, mit einer Spritze topisch appliziert wurden. Der Wirkstoff wurde durch die Cuticula absorbiert. Die Tiere wurden in einem großen Mäuseglas auf Baumwollkernen und Wasser gehalten. Nach der Adulthäutung wurden die überlebenden Tiere kontrolliert, dann wurden die Eierreproduktion und das Ausschlüpfen beobachtet.

2) Kohlraupe:

Aus 50-EC-Formulierungen wurde eine Reihe von Lösungen in Wasser mit bestimmter Konzentrationsabstufung hergestellt; dann wurden damit Kohlpflanzen tropfnaß besprüht. Nach Trocknen des Sprühbelages wurden die Pflanzen mit jeweils 20 Kohlraupenlarven im II. Stadium belegt und unter langer Beleuchtung (18 Stunden hell, 6 Stunden dunkel) gehalten. Die abgefresssenen Pflanzen wurden erforderlichenfalls durch gleich behandelte Pflanzen ersetzt.Neben der Mortalität wurden die Entwicklung der Larven bzw. eventuelle morphologische Änderungen der überlebenden Puppen und Adulten bestimmt.

3) Senfblattkäfer:

Senfpflanzen im Alter von zwei Wochen wurden mit einer Zusammensetzung aus 0,1 g Wirkstoff, 0,5 ml Dimethylsulfoxid, 10 µl 50-EC-Zusatzstoffen und 10 ml Wasser besprüht. Nach Trocknen des Sprühbelages wurden die Pflanzen mit jeweils 20 Larven von Senfblattkäfern im II. Stadium belegt. Die Pflanzen wurden alle zwei Tage durch gleichbehandelte Pflanzen ersetzt. Es wurde die Anzahl der aus den Puppen kriechenden Imagines bestimmt.

4) Stubenfliege:

Zu dem zur Zucht der Larven verwendeten Nährboden wurde in Glasphiolen in Milch gelöster Wirkstoff in einer auf den Nährboden berechneten Konzentration von 0,05 Masse-% gegeben. In die Phiolen wurden jeweils 25 Larven im I. Stadium gegeben; die Phiolen wurden verschlossen. Nach der Bildung der Puppen wurden diese herausgenommen, gezählt und bis zum Auskriechen in Petrischalen gehalten. Es wurde die Menge der ausgeschlüpften Fliegen bestimmt.

5) Caenorhabditis elegans:

Auf bakterienfreie Agarplatten wurden 0,5 ml Wirkstofflösung in Aceton aufgetragen. Nach Trocknen des Acetons wurden die Platten mit 25 bis 30 jungen Adulten belegt. Nach 24 Stunden wurde die

Anzahl der lebenden und toten Tiere bestimmt.

6) Wurzelgallennematode:

Es wurden Tests an infektiven Larven im II. Stadium durchgeführt. Man sammelte Eierbüschel, die dann auf einem Filter in sterilem Wasser bei 25 °C inkubiert wurden. Die ausgeschlüpften Larven wurden alle 24 Stunden herausgenommen und sofort in Petrischalen auf bakterienfreie Agarplatten aufgetragen. Es wurden 0,5 ml Wirkstofflösung in Aceton auf die Oberfläche der Petrischalen-Platten, dann nach Trocknen des Acetons 5 /ul Larvensuspension auf jede Platte aufgetragen. Nach 72 Stunden wurde die Anzahl der lebenden und toten Tiere bestimmt.

Die Testergebnisse sind in den Tabellen 2 bis 9 angegeben. In jeder Tabelle sind auch die Ergebnisse der Kontrollversuche aufgeführt.

Zur Kontrolle wurde das Präparat 50-EC verwendet. P1 und die Verbindung Nr. 1 wurden in einer Wirkstoffkonzentration von 0,01 %, alle anderen in einer Wirkstoffkonzentration von 0,1 % in Form des Präparates 50-EC verwendet.

7) Test des "reinen" Juvenilhormons durch Corpora allata-Tests (CA-Tests) in vitro:

Die CA von mit Kohlendioxid eingeschläferten Heuschrecken wurden herausoperiert, in Heuschrecken-Ringer-Lösung gegeben und mit dem entsprechenden Wirkstoff 24 Stunden lang inkubiert, dann mit

0,1 %-igem Acridinorange gefärbt. (Die abgestorbenen Zellen färben sich.) Der $ED_{50}$-Wert ist die Konzentration, bei der 50 % der Zellen gefärbt werden.

Tabelle 2

Wirksamkeit gegenüber Baumwollwanzen

| Wirkstoff | $LD_{50}$ (µg/Tier) | AJH-Wirkung (µg/Tier) | sterilisierende Wirkung (µg/Tier) |
|---|---|---|---|
| P2 | 0,6 | 1 | 10 |
| Verbind. 60 | 0,6 | - | - |
| Verbind. 66 | 0,4 | 0,1 | - |
| Verbind. 67 | 0,4 | - | - |
| Kontrolle+ | - | - | - |

+ = mit Aceton behandelt überlebten die Tiere zu 90 - 95 %.

Tabelle 3

Wirksamkeit gegenüber Kohlraupen

| Wirkstoff | Mortalität der Larven (%) | Überlebensrate (%) | |
|---|---|---|---|
| | | Puppen | Adulte |
| P1 | 41 | 59 | 50 |
| Verbind. 1 | 52 | 48 | 39 |
| Verbind. 17 | 81 | 19 | 19 |
| Verbind. 20 | 11 | 19 | 14 |
| Verbind. 21 | 100 | - | - |
| Verbind. 22 | 100 | - | - |
| Verbind. 30 | 83 | 17 | 17 |

(Fortsetzung von Tabelle 3)

| Wirkstoff | | Mortalität der Larven | Überlebensrate (%) Puppen | Adulte |
|---|---|---|---|---|
| Verbind. | 107 | 88 | 12 | 12 |
| " | 108 | 87 | 13 | 9 |
| " | 109 | 100 | - | - |
| " | 110 | 100 | - | - |
| " | 87 | 76 | 24 | 24 |
| " | 88 | 100 | - | - |
| " | 89 | 100 | - | - |
| " | 90 | 64 | 36 | 32 |
| " | 91 | 93 | 7 | 7 |
| Kontrolle+ | | 12 | 88 | 88 |

+ = 50-EC-Formulierung ohne Wirkstoff
Wirkstoffe P1 und Verbindung 1 in einer Konzentration von 0,01 %, die anderen von 0,1 % in 50-EC-Formulierung

Tabelle 4

Wirksamkeit gegenüber Senblattkäfern

| Wirkstoff | | Konzentration (%) | Überlebensrate Adulte (%) |
|---|---|---|---|
| Verbind. | 21 | 1 | 27 |
| " | 88 | 1 | 45 |
| Kontrolle | | 0 | 100 |

020814

Tabelle 5

Wirksamkeit gegenüber Stubenfliegen

| Wirkstoff | Überlebensrate (%) Puppen | Adulte |
|---|---|---|
| P1 | 100 | 33,3 |
| Verbind. 3 | 100 | 28,2 |
| " 17 | 40 | 14,8 |
| " 19 | 50,9 | 27,6 |
| " 20 | 25,4 | 12,5 |
| " 21 | 45,7 | 25,5 |
| " 22 | 52,7 | 31,8 |
| " 30 | 50,9 | 10,6 |
| " 107 | 9,1 | 6,4 |
| " 110 | 0 | 0 |
| " 87 | 5,4 | 4,2 |
| " 88 | 12,7 | 0 |
| " 90 | 12 | 0 |
| Kontrolle | 100 | 100 |

Tabelle 6

Wirksamkeit gegenüber Kartoffelkäfern

| Wirkstoff | Anzahl der Imagines (%) |
|---|---|
| P2 | 68 |
| Verbind. 56 | 0 |
| " 91 | 21 |
| Kontrolle | 95 |

0208147

Tabelle 7

Nematocide Wirksamkeit gegenüber Caenorhabditis elegans

| | Wirkstoff | Konzentration (/ug/ml) | Mortalität (%) | Nachkommen | Bemerkung |
|---|---|---|---|---|---|
| | P1 | 200 | 44 | reduziert | |
| | | 400 | 98 | keine | |
| | P2 | 200 | 100 | keine | |
| | | 400 | 100 | keine | |
| | P3 | 200 | 70 | einige | |
| | | 400 | 90 | keine | |
| erbind. | 30 | 200 | 20 | einige | lahm |
| | | 400 | 30 | keine | lahm |
| " | 58 | 200 | 40 | einige | |
| | | 400 | 90 | keine | |
| " | 87 | 200 | 40 | reduziert | |
| | | 400 | 100 | keine | |
| " | 90 | 200 | 60 | reduziert | |
| | | 400 | 73 | reduziert | |
| " | 89 | 200 | 25 | reduziert | |
| | | 400 | 85 | reduziert | |
| | Kontrolle | - | 0 | normal | |
| | 10 % Aceton | - | 0 | normal | |

Tabelle 8

Wirksamkeit gegenüber Kohlraupen a) und Kartoffelkäfern b)

| Verbindung von Tab.1 | Topische Behandlung ($LD_{50}$, /ug/Tier) | Blattbehandlung ($LD_{50}$, (%)) |
|---|---|---|
| a) P1 | 70 | 0,2 |
| P2 | 10 | 0,2 |
| P3 | 30 | 0,1 |
| 30 | 10 | 0,15 |
| 50 | 5 | 0,05 |
| 107 | 3 | 0,1 |

(Fortsetzung von Tabelle 8

| Verbindung von Tabelle 1 | Topische Behandlg. ($LD_{50}$, µg/Tier) | Blattbehandlung ($LD_{50}$, (%) |
|---|---|---|
| b) P1 | 88 | 0,2 |
| P2 | 12 | 0,2 |
| 50 | 5 | 0,01 |
| 107 | 5 | 0,08 |

Tabelle 9

In-vitro-CA-Tests zur direkten Messung der Juvenilhormon-Wirkung

| Wirkstoff | $EC_{50}$ (µmol/ml) |
|---|---|
| P1 | 43,33 |
| P2 | 58,71 |
| Verbind. 4 | 26,07 |
| " 68 | 38,49 |
| " 18 | 33,24 |

Neuanmeldung (= Teilanmeldung aus 49-35.862)

## Patentansprüche

1. 2H-Chromenderivate der allgemeinen Formel I

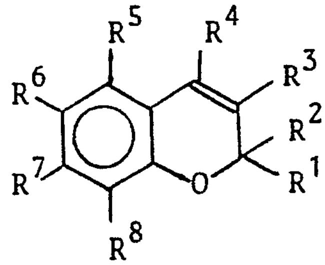

(I),

worin bedeuten:

$R^1$ und $R^2$ Wasserstoff oder $C_{1-4}$-Alkyl,

$R^3$ und $R^4$ Wasserstoff oder Halogen,

$R^5$ Wasserstoff, $C_{1-10}$-Alkyl oder $C_{1-6}$-Alkoxy,

$R^6$ Wasserstoff, $C_{1-6}$-Alkoxy, das gesättigt oder ungesättigt und gegebenenfalls mit einer Trihalogenmethylgruppe substituiert ist, $C_2$- oder $C_3$-Alkylendioxy oder Aralkyloxy,

$R^7$ $C_{2-3}$-Alkoxy, das gesättigt oder ungesättigt und gegebenenfalls mit einer $C_{1-6}$-Alkyl-, $C_{1-6}$-Alkylthio-, $C_{1-6}$-Alkoxy-, Trihalogenmethyl- oder Hydroxylgruppe substituiert ist, $C_{5-8}$-Cycloalkyloxy oder Aralkyloxy, das gegebenenfalls mit einem Halogenatom substituiert ist, Dialkylaminoalkoxy

oder Cycloalkylaminoalkyloxy,

wobei $R^6$ und $R^7$ zusammen auch $C_{2-8}$-Alkylenoxy-alkylenoxy bedeuten können,

und

$R^8$ Wasserstoff, $C_{1-6}$-Alkoxy oder Aralkoxy oder $C_{1-6}$-Alkyl,

wobei $R^7$ und $R^8$ zusammen auch $C_{2-8}$-Alkylenoxy-alkylenoxy bedeuten können,

mit der Maßgabe, daß, wenn $R^6$ Methoxy und $R^7$ Ethoxy- oder Isopropyloxy darstellen, $R^3$, $R^4$ und $R^5$ nicht gleichzeitig Wasserstoff bedeuten können,

und ihre Salze.

2. Verfahren zur Herstellung der 2H-Chromenderivate der Formel I nach Anspruch 1,

dadurch gekennzeichnet, daß

(A) zur Herstellung von 2H-Chromenderivaten der allgemeinen Formel I mit $R^3$, $R^4$ = H, d.h. von 2H-Chromenderivaten der Formel IA,

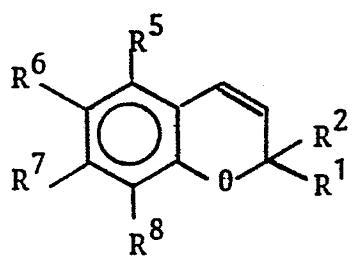

(IA),

worin bedeuten:

$R^1$ und $R^2$ Wasserstoff oder $C_{1-4}$-Alkyl,

$R^3$ und $R^4$ Wasserstoff oder Halogen,

$R^5$ Wasserstoff, $C_{1-10}$-Alkyl oder $C_{1-6}$-Alkoxy,

$R^6$ Wasserstoff, $C_{1-6}$-Alkoxy, das gesättigt oder ungesättigt und gegebenenfalls mit einer Trihalogenmethylgruppe substituiert ist, $C_2$- oder $C_3$-Alkylendioxy oder Aralkyloxy,

$R^7$ $C_{2-3}$-Alkoxy, das gesättigt oder ungesättigt und gegebenenfalls mit einer $C_{1-6}$-Alkyl-, $C_{1-6}$-Alkylthio-, $C_{1-6}$-Alkoxy-, Trihalogenmethyl- oder Hydroxylgruppe substituiert ist, $C_{5-8}$-Cycloalkyloxy oder Aralkyloxy, das gegebenenfalls mit einem Halogenatom substituiert ist, Dialkylaminoalkoxy oder Cycloalkylaminoalkyloxy,

wobei $R^6$ und $R^7$ zusammen auch $C_{2-8}$-Alkylenoxy-alkylenoxy bedeuten können,

und

$R^8$ Wasserstoff, $C_{1-6}$-Alkoxy oder Aralkoxy oder $C_{1-6}$-Alkyl,

wobei $R^7$ und $R^8$ zusammen auch $C_{2-8}$-Alkylenoxy-alkylenoxy bedeuten können,

mit der Maßgabe, daß, wenn $R^6$ Methoxy und $R^7$ Ethoxy- oder Isopropyloxy darstellen, $R^3$, $R^4$ und $R^5$ nicht gleichzeitig Wasserstoff bedeuten können,

und ihrer Salze,

eines der folgenden Verfahren angewandt wird:

(a) Dehydratisierung von Chromanolderivaten der allgemeinen Formel

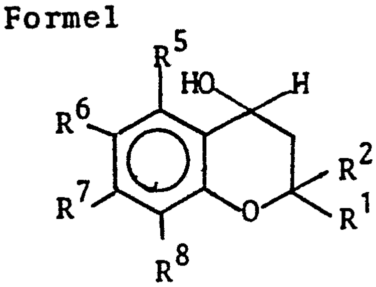

(III)

mit $R^1$, $R^2$ und $R^5$ - $R^8$ wie oben;

(b) Umsetzung von Cumarinderivaten der Formel IV

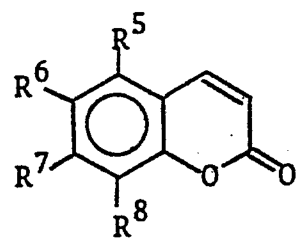

(IV)

mit $R^5$ - $R^8$ wie oben

mit Grignard-Reagentien der Formel

$R^1MgX$ und/oder $R^2MgX$,

worin $R^1$ und $R^2$ die obige Bedeutung besitzen und

X Halogen darstellt;

(c) Dehydratisierung von Chinonalliden der allgemeinen Formel VI

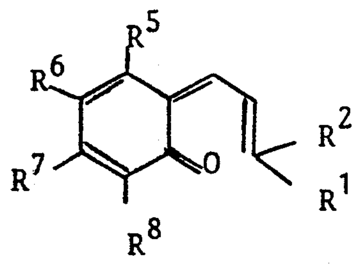

(VI)

mit $R^1$, $R^2$ und $R^5$- $R^8$ wie oben,

vorzugsweise in aromatischen Lösungsmitteln bei 60 bis 120 °C mit Kaliumdichromat;

(d) Dehydrohalogenierung von Chromanderivaten der allgemeinen Formel VII

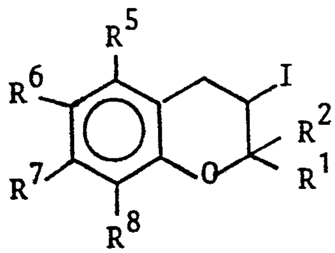

(VII)

mit $R^1$, $R^2$ und $R^5$ - $R^8$ wie oben,

vorzugsweise in alkoholisch/basischem Medium beim Siedepunkt der Alkohole;

(e) Cyclisierung von Ether der allgemeinen Formel X

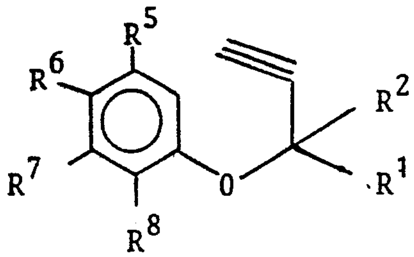

(X)

mit $R^1$, $R^2$ und $R^5$ - $R^8$ wie oben,

vorzugsweise in N,N-Dialkylarylaminen bei 160 bis 250 °C;

(f) Umsetzung von Phenolen der allgemeinen Formel VIII

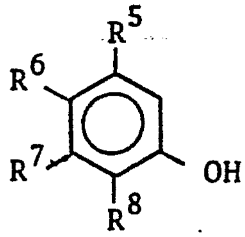

(VIII)

mit $R^5$ - $R^8$ wie oben

mit Carbonylverbindungen der allgemeinen Formel XI

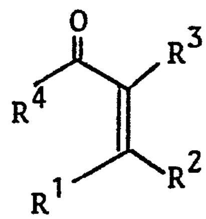

(XI)

mit $R^1$ - $R^4$ wie oben

oder deren geschützten Derivaten, vorzugsweise in aromatischen aprotischen Lösungsmitteln in Form ihrer entsprechenden Schwermetallsalze bei 100 bis 150 °C;

(g) Oxidation von Chromanen der allgemeinen Formel XIII

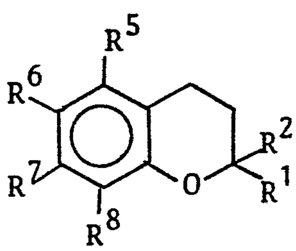

(XIII)

mit $R^1$, $R^2$ und $R^5$ - $R^8$ wie oben;

020814

(B) zur Herstellung von 2H-Chromenderivaten der allgemeinen Formel I mit $R^3$, $R^4$ = Halogen, d.h. von 2H-Chromenderivaten der allgemeinen Formel IB

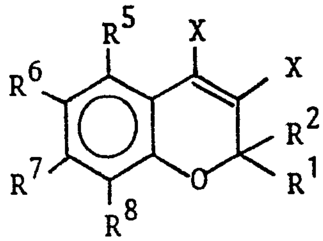

(IB)

mit $R^1$, $R^2$ und $R^5$ - $R^8$ wie oben
und
X = Halogen,

(h) 4-Chromanonderivate der allgemeinen Formel II

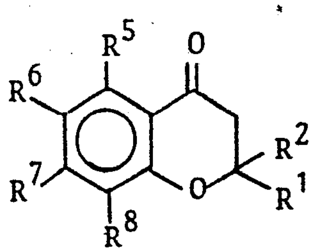

(II)

mit $R^1$; $R^2$ und $R^5$ - $R^8$ wie oben

mit Phosphorpentahalogeniden, vorzugsweise in Kohlenstofftetrachlorid bei 15 bis 25 °C, umgesetzt werden, und anschließend aus der erhaltenen Verbindung ein Salz gebildet oder aus dem Salz die Verbindung der Formel IB freigesetzt wird.

3. Verfahren nach Anspruch 2, dadurch gekennzeichnet, daß beim Verfahren (a) die Chromanolderivate der allgemeinen Formel III mit einem Dehydratisierungsmittel bei 20 bis 160 °C, vorzugsweise mit verdünnter Salzsäure

bei 15 bis 25 °C, dehydratisiert werden.

4. Verfahren nach Anspruch 2, dadurch gekennzeichnet, daß beim Verfahren (b) Dimethylether oder Tetrahydrofuran als Lösungsmittel verwendet werden, und die Umsetzung bei 35 bis 65 °C durchgeführt wird.

5. Verfahren nach Anspruch 2, dadurch gekennzeichnet, daß beim Verfahren (e) die Cyclisierung in N,N-Dimethylanilin oder N,N-Diethylanilin bei 190 bis 210 °C durchgeführt wird.

6. Verfahren nach Anspruch 2, dadurch gekennzeichnet, daß beim Verfahren (f) die Titansalze der Phenole der allgemeinen Formel XI eingesetzt werden, und die Umsetzung in siedendem Toluol durchgeführt wird.

7. Verfahren nach Anspruch 2, dadurch gekennzeichnet, daß beim Verfahren (g) die Chromanderivate der allgemeinen Formel XIII in aromatischen Lösungsmitteln bei 60 bis 150 °C oxidiert werden.

8. Verfahren nach Anspruch 2 oder 7, dadurch gekennzeichnet, daß beim Verfahren (g) die Oxidation mit Dichlordicyanobenzochinon (DDQ) in Benzol bei 50 bis 80 °C durchgeführt wird.

9. Verfahren nach Anspruch 2, dadurch gekennzeichnet, daß beim Verfahren (h) die 4-Chromanonderivate der allgemeinen Formel II in Halogenalkanen oder Perhalogenalkanen bei 10 bis 50 °C mit Phosphorpentahalogeniden umgesetzt werden.

10. Verfahren nach Anspruch 2 oder 9, dadurch gekennzeichnet, daß beim Verfahren (h) als Phosphorpentahalogenid Phosphorpentachlorid eingesetzt wird.

11. Schädlingsbekämpfungsmittel, gekennzeichnet durch einen Gehalt an 2H-Chromenen der Formel I nach Anspruch 1 als Wirkstoffen neben üblichen festen Trägerstoffen und/oder flüssigen Verdünnungsmitteln und gegebenenfalls grenzflächenaktiven Stoffen.